# EUROPEAN PATENT APPLICATION

(11) **EP 2 267 144 A2**
(43) Date of publication of application: **29.12.2010**
(21) Application number: 10188509.3
(22) Date of filing: 04.12.2007
(51) Int. Cl.: C12P 13/04, C12P 13/22, C12P 41/00, C12P 17/10, C07C 229/30, C07C 229/32, C07D 233/74, C07D 233/76

(54) **Whole-cell catalytic system comprising a hydantoinase, a racemase and a carbamoylase**

(62) Divisional of application: 07856353.3
(71) Applicant: DSM IP Assets B.V., 6411 TE Heerlen (NL)
(72) Inventor: Rusnak-Müller, Monika, 52070, AACHEN (DE); May, Oliver, 52076, AACHEN (DE); Hermsen, Petrus Johannes, 5961 VC, Horst (NL); Straatman, Henricus Martinus Maria Gerardus, 5961 LG, Horst (NL); Skranc, Wolfgang, A-1220, VIENNA (AT); Boesten, Wilhelmus Hubertus Joseph, 6132 BJ, SITTARD (NL); Heemskerk, Dennis, 6451 AD, SCHINVELD (NL); De Lange, Ben, 6151 GE, MUNSTERGELEEN (NL); Sarakinos, Georgios, 80796, MUNICH (DE)
(74) Representative: Hoogendam, Gerrie Christine

(57) **Abstract**

The present invention relates to a whole cell catalytic system for the preparation of an enantiomerically enriched a-amino acid from a corresponding hydantoin wherein hydantoinase, L-carbamoylase and hydantoin racemase are coexpressed in a recombinant micro-organism wherein the genes coding for these three enzymes are located on a single replicon.

The present invention further relates to the use of such a whole cell catalytic system according in the preparation of an enantiomerically enriched L-α-amino acid from a corresponding hydantoin.

## Description

The present invention relates to a whole cell catalytic system for the preparation of an enantiomerically enriched α-amino acid from a corresponding hydantoin and to the use of such whole cell catalytic system for said conversion.

Whole cell catalytic systems for the preparation of an enantiomerically enriched α-amino acid from a corresponding hydantoin are known from WO01/23582. According to WO01/23582 *E. coli* has been equipped with genetic material encoding the three enzymes hydantoinase, carbamoylase and hydantoin racemase of *Arthrobacter aurescens* DSM 3747 and the genes are overexpressed in the cells according to their turnover rates. This was done in order to "fine tune" the expression of the genes. More in particular this publication discloses the expression of the three genes from the same promoter but from separate replicons with different copy numbers.

The present invention relates to an improvement of such whole cell catalytic system for the preparation of an enantiomerically enriched L-α-amino acid from a corresponding hydantoin. According to the present invention hydantoinase, L-carbamoylase and hydantoin racemase are coexpressed in a recombinant micro-organism wherein the genes coding for these three enzymes are located on a single replicon.

According to a further embodiment of the present invention the hydantoin racemase is derived from a gene of an *Agrobacterium* species.

In a preferred amodiment, the hydantoin racemase is derived from a gene of an *Agrobacterium* species and the hydantoinase and L-carbamoylase are each independently derived from genes from species other than an *Agrobacterium* species. Suitbale species to derive genes for the hydantoinase and L-carbamoylase from are for example Arthrobacter, Pseudemonas and Bacillus.

Such a whole cell catalytic system is particularly useful for the preparation of an enantiomerically enriched L-α-amino acid starting from a substrate with the general formula [**1**] 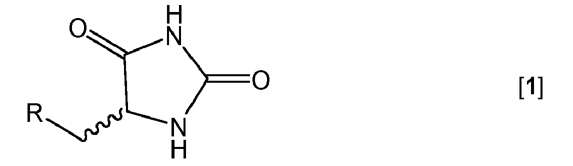 wherein R denotes a substituent with at least 3 carbon atoms, optionally containing further one or more hetero atoms, one or more double bonds and/or one or more cyclic structures. It is an advantage of the method according to the invention, that both enantiomers of the compound according to formula [1] can be converted in the desired S-amino acid.

This particular combination of genes on a single replicon has never been applied to the preparation of an enantiomerically enriched L-α-amino acid from a corresponding hydantoin, and it was surprising that such conversion was possible at a high yield even without fine tuning of the copy numbers as described to be necessary according to the prior art cited above.

Suitably the three genes encoding the respective enzymes hydantoinase, L-carbamoylase and hydantoin racemase are under the control of a single promoter. Promoters useful according to the present invention are promoters suited for expression of genes in the particular recombinant micro-organism. Examples of such promoters are inducible promoters for operons/genes like the lactose operon (*lac*), the rhamnose operon (rha), the arabinose operon (ara), the tryptophan operon (*trp*), the operon encoding enzymes common to the biosynthesis of all aromatic amino acids (*aro*), or functional hybrids of these. Other examples of useful promoters are constitutive promoters.

According to a further embodiment of the present invention the single replicon contains SEQ ID NO: 2 encoding L-hydantoinase, SEQ ID NO: 3 encoding L-carbamoylase and SEQ ID NO: 4 encoding hydantoin racemase.

It will be obvious to a person skilled in the art that whenever in this text sequences are identified, the invention also relates to all sequences with a homology of 90%, more preferably 95%, 96%, 97%, 98% or 99% to the identified sequence. For the purpose of this invention, homology is determined according to the procedure described by Tatiana A. Tatusova and Thomas L. Madden "Blast 2 sequences - a new tool for comparing protein and nucleotide sequences", FEMS Microbiol Lett. 174:247-250 (1999), The following are the standard parameters used at http://www.ncbi.nlm.nih.gov/BLAST/bl2seq/wblast2.cgifor Protein sequences:
Matrix: BLOSUM62
Open gap: 5
extension gap: 2
Penalties gap x_dropoff: 11
Expected: 10
word size: 11

DNA Sequences with a homology of 90%, more preferably 95%96%, 97%, 98% or 99% to the DNA sequences according to the invention are explicitly also part of the invention for the DNA constructs according to the invention. The following standard parameters are thereby used at
http://www.ncbi.nlm.nih.gov/BLAST/bl2seq/wblast2.cgifor Protein sequences: Reward for match:1
Penalty for mismatch:-2
Open gap: 11
Extension gap: 1
Penalties gap x_dropoff: 50
Expected: 10
Word size: 3

The present invention also relates to a DNA construct containing the sequences encoding hydantoinase, L-carbamoylase and hydantoin racemase operationally linked to a single promoter. More in particular the invention relates to a DNA construct wherein the sequence encoding hydantoin racemase is derived from an *Agrobacterium* species. More specifically the DNA construct may contain the sequences encoding hydantoinase of the sequence represented by SEQ ID NO: 2, carbamoylase of the sequence represented by SEQ ID NO: 3 and racemase of the sequence represented by SEQ ID NO: 4.

In a further embodiment the present invention relates to an expression vector containing a DNA construct described above.

The DNA sequences encoding the respective enzymes may be the sequences occurring naturally, or may be synthetic sequences. The synthetic sequences may be adapted compared to the naturally occurring sequences e.g. by using codons for the amino acids which are more suitable for expression in the particular recombinant microorganism selected for the whole cell catalytic systems of the present invention.

The replicon for use according to the present invention can be a plasmid, a phage or a chromosome. The replicon may comprise a marker enabling selection of cells harboring the element, as well as DNA sequences responsible for autonomous propagation and or equal distribution of the element within the host cell and its daughter cells.

Suitable microorganisms for use according to the invention are prokaryotes and in particular bacteria, and more in particular *E*. *coli.*

The present invention also relates to the use of a whole cell catalytic system as described above in the preparation of enantiomerically enriched α-amino acids from a corresponding hydantoin.

In particular, the invention relates to the use of said whole cell catalytic system for the preparation of an enantiomerically enriched L-α-amino acid of general formula **[2]** 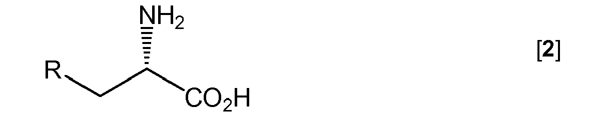 starting from a substrate with the general formula [**1**] 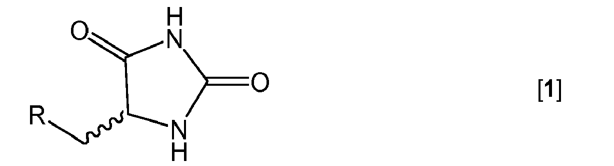 wherein R has the meaning given above.

More in particular the invention also relates to the use of the whole cell catalytic system in the conversion of a hydantoin of formula **[3]** 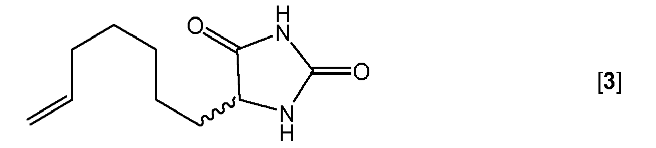 into an L-α-amino acid of formula **[4]** 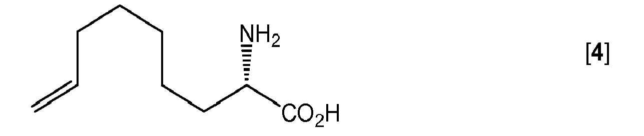 wherein R has the meaning given above.

The present invention also concerns compounds of formula **[3]** and **[4].**

Also, the invention relates to the use of the whole cell catalytic system in the conversion of a hydantoin of formula **[5a]** or **[5b]** 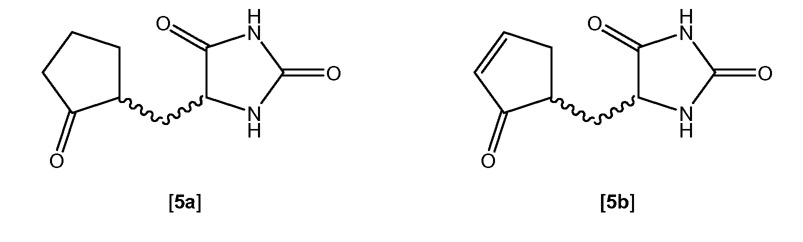 into an L-α-amino acid of formula **[6a]** or **[6b]** respectively 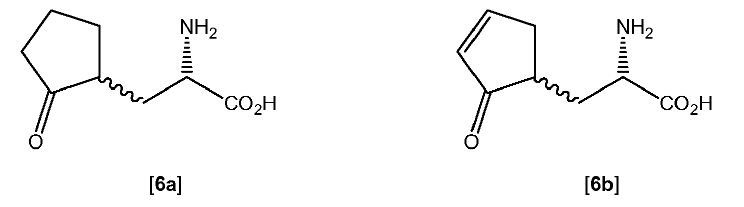 L-α-amino acids of formula [**6a**] and [**6b**] can be used as intermediates in the synthesis of the angiotensin converting enzyme (ACE) inhibitor ramipril. In most prior art methods ramipril is prepared starting from racemic **[6a],** requiring a subsequent optical resolution that inevitably results in loss of approximately 50% of the end-product, a problem that is solved by using L-α-amino acid of formula **[6a]** or **[6b].** Thus, in one embodiment a method is presented for the conversion of L-α-amino acid of formula **[6a]** or **[6b]** by hydrogenation into (2S,3aS,6aS)-octahydrocyclopenta[b]pyrrole-2-carboxylic acid, which is a building block common in all commercially viable routes towards ramipril known today. In a preferred embodiment, hydrogenation of L- α-amino acid of formula **[6a]** or **[6b]** is carried out using hydrogen gas in the presence of a suitable catalyst such as for example palladium on charcoal. Furthermore, the invention relates to the use of the whole cell catalytic system in the conversion of a hydantoin of formula **[7a]** or **[7b]** 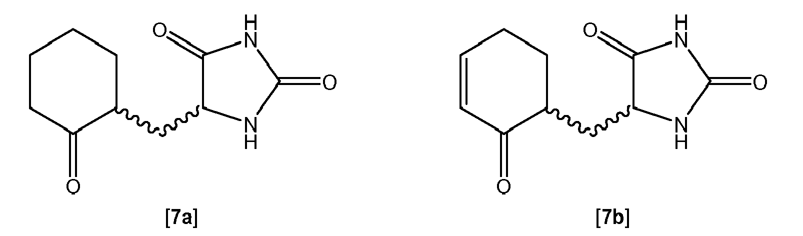 into an L-α-amino acid of formula **[8a]** or **[8b]** respectively 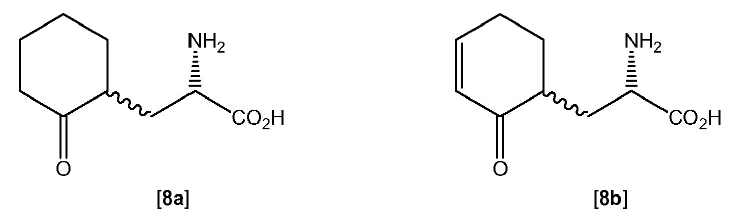 L-α-amino acids of formula **[8a]** and **[8b]** can be used as intermediates in the synthesis of the angiotensin converting enzyme (ACE) inhibitor perindopril, with similar advantages as described above for L-α-amino acids of formula **[6].** Thus, in one embodiment a method is presented for the conversion of L-α-amino acid of formula **[8a]** or **[8b]** by hydrogenation into
(2S,3aS,6aS)-decahydrocyclohexa[b]pyrrole-2-carboxylic acid, which is a building block common in all commercially viable routes towards perindopril known today.
Also, the invention relates to the use of the whole cell catalytic system
in the conversion of a hydantoin of formula **[9]** 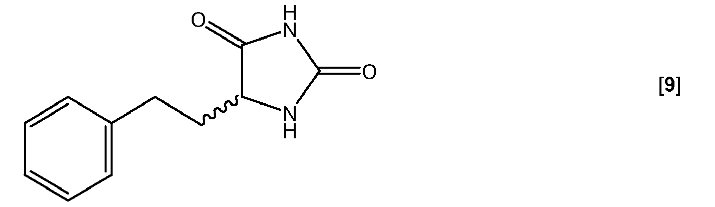 into an L-α-amino acid of formula **[10]**

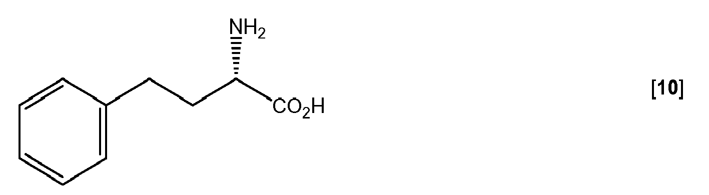

The compounds of formula **[5], [6], [7]** and **[8]** are novel compounds and are also part of the present invention.

In the present context "enantiomerically-enriched" means that one enantiomer or set of diastereomers preponderates over the complementary enantiomer or set of diastereomers.

In the present context "amino acid" means a compound in which at least one amino group and at least one carboxyl group are bound to the same carbon atom (α-C-atom)

### Description of the Figures

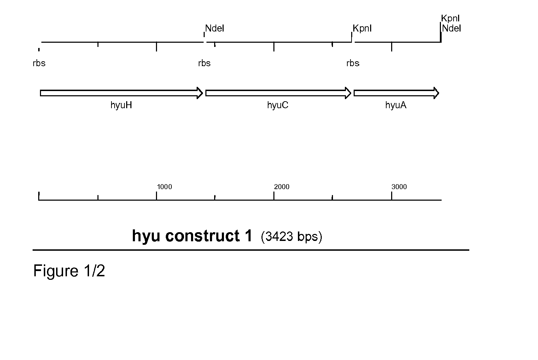

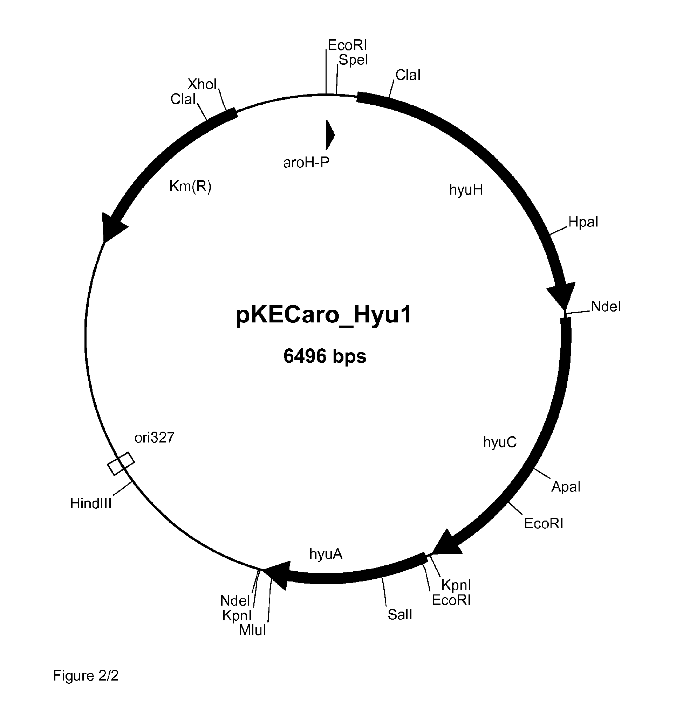

- Fig. 1.: A physical and functional map representing the basic construction of the operon (Hyu1 Operon). hyuH, hyuC and hyuA represent the genes coding for L-hydantoinase from *Arthrobacter aurescens,* L-carbamoylase from *Bacillus stearothermophilus* and hydantoin racemase from *Agrobacterium radiobacter* (HyuA), respectively. rbs means ribosomal binding site. Ndel and Kpnl indicate restriction sites.
- Fig. 2.: A physical and functional map representing the basic construction of the expression vector pKECaro_hyu1. hyuH, hyuC and hyuA have the meanings described above. Km(R) is the kanamycin resistance gene. ori327 is the origin of replication from plasmid pBR327. aroH-P is the promoter of tryptophan- sensitive 3-deoxy-D-arabinoheptulosonic acid-7-phosphate synthetase. Clal, Spel, Hpal, Ndel, Apal, EcoRI, Kpnl, SaII, Mlul, Hindlll and Xho1 indicate restriction sites.

### Materials and methods

Unless indicated otherwise, all molecular techniques employed were essentially performed according to Maniatits *et al.* (J. Sambrook, E.F. Fritsch, T. Maniatis. Molecular Cloning 2nd edition. CSH Press).

### Protocol for transformation of pKECaroP-hyu1 construct into E.coli RV308

- Thaw *E.coli* RV308 aliquots (200 µl, supercompetent) on ice
- Add 15 µl LR reaction mix (see above)
- Incubate 30 minutes on ice
- Heatshock 1 minute 42°C
- Cool cells 2 minutes on ice
- Add 1 ml LB medium (5 g/I NaCl, 5 g/I yeast extract, 10 g/I tryptone)
- Incubate 1 hour 37°C
- Plate on LB agar plates supplemented with kanamycine (5 g/I NaCl, 5 g/I yeast extract, 10 g/I tryptone, 15 g/I agar, 50 mg/l kanamycine)
- Incubate 24 hours 28°C
- Isolate single colonies

### Protocol for expression of Hyu genes in E.coli RV308

Single clones from the transformation (see above) were used to inoculate 5 ml of 2xTY media (10g/l yeast extract, 16g/l tryptone, 5g/l NaCl) supplemented with 0.05g/l kanamycine and 1 mM MnCl₂ or CoCl₂, respectively. The culture was incubated at 28°C and 150 rpm for 24 hours and then used for inoculation of 100 ml 2xTY media supplemented with 0.05 g/I kanamycine and 1 mM MnCl₂ or CoCl₂, respectively. The cultures were again incubated for 24 - 28 hours under conditions previously mentioned and subsequently harvested by centrifugation (20 minutes, 5000 rpm, 4°C). The cell pellet was resuspended in 5 ml Tris-HCl (100 mM, pH 7), centrifuged again (20 minutes, 5000 rpm, 4°C) and the cells were frozen at -20°C.

### Examples

### Example 1. Construction of a clone for co-expression of L-Hydantoinase, L-Carbamoylase and Hydantoin Racemase in E.coli RV308

The aim was to obtain active coexpression of the L-hydantoinase from *Arthrobacter aurescens* (HyuH), the L-carbamoylase from *Bacillus stearothermophilus* (HyuC) and the hydantoin racemase from *Agrobacterium radiobacter* (HyuA) in the host *E. coli* RV308 resulting in a production strain for the production of L-amino acids.

The sequences of the 3 enzymes are known from the following literature sources:
■ L-hydantoinase (represented below as SEQ ID NO: 2) from Abendrodt et al. Biochemistry 41 (27), 8589-8597 (2002)
■ L-carbamoylase (represented below as SEQ ID NO: 3) from Battise et al. Appl. Environ.Microbiol. 63(2), 763-766 (1997
■ Hydantoin racemase (represented below as SEQ ID NO: 4) from patent EP 1506294B1,

An operon (represented by SEQ ID NO: 1) was synthetically prepared wherein the three genes of the hydantoin pathway (hyuH, hyuC, hyuA) are separated from each other by spacers containing a ribosomal binding site rbs (Shine-Delgarno Sequence) and a restriction site for further subcloning.

The order of the subsequences in the construct is as follows:
■ Restriction site 1 (SEQ ID NO: 5);
■ Sequence coding for L-hydantoinase (SEQ ID NO: 2);
■ Spacer (SEQ ID NO: 6);
■ Sequence coding for L-carbamoylase (SEQ ID NO: 3);
■ Spacer (SEQ ID NO: 6);
■ Sequence coding for hydantoin racemase (represented below as SEQ ID NO 4)
   and
■ Rbs (SEQ ID NO: 7).

A map representing the basic construction of the operon (Hyu1 Operon) is illustrated in Fig. 1.

The DNA sequences of the enzyme-encoding regions were optimized for expression in *E.coli* RV308.

The Hyu1 operon was subsequently cloned into an expression vector. The expression vector pKECaro_hyu1 is derived from plasmid pKECtrp (described in WO 0066751) by replacing the trp promoter ==> PenG acylase expression cassette by the aroH promoter ==> hyu1 operon.

The DNA was transformed into supercompetent *E.coli* RV308 cells (as described in Material and Methods) and single clones were isolated from the agar plate. The clones were grown in LB medium supplemented with kanamycin (5 g/I NaCl, 5 g/I yeast extract, 10 g/I tryptone, 50 mg/l kanamycin) and plasmid DNA was isolated using the Qiagen Miniprep Kit (following the standard procedure). The accuracy of the constructs was checked by restriction analysis.

### Example 2. Preparation of S-2-amino-non-8-enoic acid

### Synthesis of 5-hept-6-enyl-imidazolidin-2,4-dione [3]

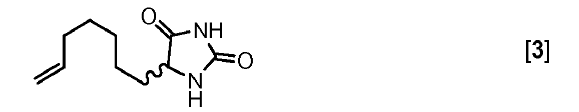

To a solution of 100,0 g ammonium carbonate and 29.4 g sodium cyanide in 950 ml of water and 300 ml of ethanol was added 63,0 g of 7-octenal. The two-phase system was heated to 60 °C and subsequently stirred overnight.

The mixture was allowed to cool to ambient temperature after which the precipitated white product was isolated by filtration, washed with water and subsequently dried *in vacuo.* The obtained crude product was purified by stirring in chloroform for 1 hour at ambient temperature. The hydantoin was isolated by filtration, washed with chloroform and dried *in vacuo* to yield 39.0 g of white solid. ¹H NMR (300 MHz, DMSO-d6) δ ppm) :10.62 (1H, NH), 7.92 (1H, NH), 5.81 (1H, m), 4.99 (2x1H, md, *J* = 18.0 Hz, 9.0 Hz), 3.98 (1H, m), 2.01 (2H, m), 1.68 (1H, m), 1.53 (1H,m), 1.35 (6H, m). ¹³C-NMR (75 MHZ, DMSO-d6) δ ppm): 176.59, 157.99, 139.07, 115.06, 57.91, 33.38, 31.55, 28.85, 28.48, 28.41, 24.32, 22.41.

It should be noted that when the starting material 7-octenal is not 100% pure but also comprises amounts of octenal and 6-octenal, the hydantoin and amino acid derivates thereof are also produced.

### Synthesis of S-2-amino-non-8-enoic acid [4]

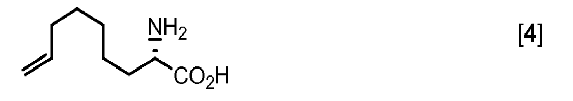

1.9 g of wet cells obtained according to the above *Protocol for expression of Hyu genes in E.coli RV308* was suspended in 40 g Tris.HCl buffer (pH 8.5). Subsequently, 450 µl of a 100 mmol/l MnCl₂ solution and 1.0 g of 5-hept-6-enyl-imidazolidin-2,4-dione were added and the mixture was incubated overnight at 37 °C. The mixture was cooled to 20 °C after which the pH was raised to 13 by the addition of 5 mol/I aqueous KOH. The cells were removed by centrifugation after which the supernatant was acidified to pH 6 by the addition of 4 N aqueous HCl. The precipitated product was isolated by filtration and subsequently washed with water and methanol to yield 0.8 g of off-white product (85% chemical yield), ee > 99.5% (HPLC). ¹H NMR (300 MHz, D₂O) δ ppm) : 5.56 (1H, tdd, *J* = 18.0 Hz, 9.0 Hz), 4.82 (1H, md, *J* = 18.0 Hz), 4.65 (1H, md, *J* = 9.0 Hz), 3.77 (1H, m), 1.71 (2H, m), 1.62 (2H, m), 1.05 (6H, m).

### Example 3. Preparation of S-phenyl alanine

0.28 g of wet cells was suspended in 3 ml of Tris.HCl buffer (pH 8.5). Subsequently, 30 µl of a 100 mM MnCl₂ solution and 57.5 mg g of 5-benzyl-imidazolidin-2,4-dione were added and the mixture was incubated overnight at 37 °C. The mixture was cooled to 20 °C after which the sample was analyzed by chiral HPLC. The S-phenyl alanine thus prepared had an ee > 99.8%.

### Example 4. Preparation of (S)-2-amino-3-(2-oxocyclopentyl)propanoic acid

### 1. Synthesis of [5a]

L-Serine hydantoin (5-hydroxymethylimidazolidin-2,4-dione, 41.4 g, 318 mmol) was suspended in dry MeCN (330 mL) and the solution was cooled to 10°C. Pyridine (88.3 g, 1.11 mol) was then added and a homogeneous solution formed. Acetyl chloride (87.4 g, 1.11 mol) was added via addition funnel drop wise during ca. 1 h, so that the reaction temperature was maintained at 10 °C. After the completion of the addition, the reaction was allowed to warm up to 20 °C (within ca. 30 min) while a precipitate formed. Slowly the reaction mixture became homogeneous again. After approx. 16 h, the reaction was complete (as determined by ¹H NMR in CDCl₃). It was then concentrated on the rotavapor at 40°C to a final weight of ca. 240 g. Some solids formed. MTBE (500 mL) was then added and more solid precipitated. After stirring for 10 min, the precipitate (pyridinium chloride) was collected on a funnel via suction. The cake was washed with additional MTBE (100 mL) and then discarded. The product, tris-acetyl 5-hydroxymethylimidazolidin-2,4-dione, resided in the mother liquor. This was concentrated on a rotavapor at 40°C to a thick oil and used as such in the next step. Weight 73.0 g, ca. 95% purity, approx. 85% yield. ¹H NMR: (CDCl₃, 300 MHz): δ 4.83 (dd, 1H), 4.73 (t, 1H), 4.44 (dd, 1H), 2.61 (s, 3H), 2.60 (s, 3H), 2.03 (s, 3H).

Tris-acetyl 5-hydroxymethylimidazolidin-2,4-dione obtained above (6.06 g, 95% pure, 22.5 mmol) was dissolved in THF (25 mL) and added drop wise via an addition funnel into a solution of triethylamine (3.57 g, 35.3 mmol) and 1-pyrrolidino-1-cyclopentene (4.85 g, 35.3 mmol) in THF (25 mL) at 20 °C over a period of approx. 45 min. The homogeneous, yellow-orange reaction was stirred further for 16 h at 20°C. The conversion was complete as determined by ¹H NMR in DMSO-d₆. Then, water (8 mL) was added followed by conc. aq. HCl (3 mL). After stirring for 30 min, the solvent was removed on the rotavapor at 40°C. Additional water (15 mL) was added and the product was extracted into EtOAc (2 x 25 mL) and the combined organic extracts were dried over anhydrous Na₂SO₄. After filtration and concentration of the solution on the rotavapor at 60°C, the product, N₄-acetyl-[**5a**] was purified by silica gel flash chromatography using an EtOAc:petroleum benzene gradient. Weight 4.35 g, 81% yield. The product N₄-acetyl-[**5a**] is a ca. 1:1 mixture of diastereomers. ¹H NMR: (CDCl₃, 300 MHz): δ 8.49 (br s, 1H), 4.82 (dd, 0.5H), 4.63 (dd, 0.5H), 2.57 (s, 3H), 2.46-2.00 (m, 7H), 1.88-1.75 (m, 1H), 1.67-1.53 (m, 1H).

N₄-acetyl-[**5a**] (0.500 g, 2.10 mmol) was dissolved in 6M aq. HCl and was stirred at 80°C for 16 h. The conversion was followed by TLC on silica-coated plates (eluent is 7:3 EtOAc:petroleum benzene). The reaction mixture was then cooled to 20°C and extracted with ethyl ether (10 mL). The organic layer was discarded. The aqueous phase was concentrated on the rotavapor at 70 °C to give 5-((2-oxocyclopentyl)methyl)imidazolidine-2,4-dione **[5a]** (mixture of diastereomers) as a brownish solid. Weight 0.360 g, 87% yield. ¹H NMR: (DMSO-d₆, 300 MHz): δ 10.63 (br s, 1H), 7.99 (br s, 0.73H, major diastereomer), 7.90 (br s, 0.27H, minor diastereomer), 4.19 (td, 0.27H, minor diastereomer), 4.06 (ddd, 0.73H, major diastereomer), 2.30-2.02 (m, 4 H), 1.98-1.80, 2 H), 1.77-1.62 (m, 1H), 1.60-1.40 (m, 2H).

### 2. Synthesis of (S)-2-amino-3-(2-oxocyc/openty/)propanoic acid [6a]

5-((2-oxocyclopentyl)methyl)imidazolidine-2,4-dione **[5a]** (0.20 g, 1.0 mmol) was suspended in TRIS-HCl buffer (400mM, 5 mL) at pH 8.5. Then, 0.8 g of wet cell slurry obtained according to 'Protocol for expression of Hyu genes in *Escherichia coli* RV308' in 'Materials and methods' was added, followed by 50 µL of a 100 mmol/L MnCl₂ solution and the mixture was incubated overnight at 37°C. The mixture was then centrifuged at room temperature and the supernatant was filtered through a 0.45 µ filter. The product aminoacid **[6a]** has the S configuration at C2 with >99% ee (the other chiral center C4 is scrambled).

### Example 5. Preparation of (S)-2-amino-3-(2-oxocyclohexyl)propanoic acid

### 1. Synthesis of [7a]

Compound **[7a]** was prepared similarly, with similar yield, as described above for compound **[5a]** however using 1-pyrrolidino-1-cyclohexene rather than 1-pyrrolidino-1-cyclopentene.

### 2. Synthesis of (S)-2-amino-3-(2-oxocyc/ohexy/)propanoic acid [8a]

Compound **[8a]** was prepared from **[7a]** similarly, with similar yield, as described above for the synthesis of compound **[6a]** from compound **[5a].**

## Claims

1. A compound according to formula [3]

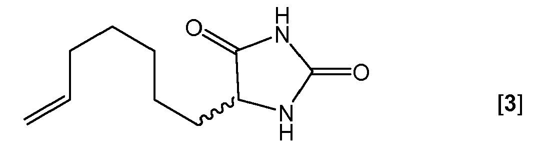

2. A compound according to formula [4].

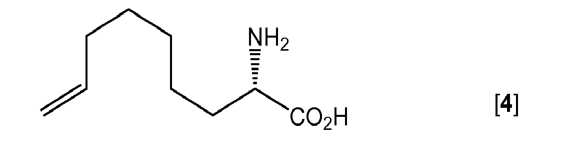

3. Use of a whole cell catalytic system for the preparation of an enantiomerically enriched L- α-amino acid from a corresponding hydantoin wherein in the whole cell catalytic system a hydantoinase, L-carbamoylase and hydantoin racemase are coexpressed in a recombinant micro-organism wherein the genes coding for these three enzymes are located on a single replicon, and wherein the hydantoin racemase is derived from an *Agrobacterium* species, and wherein the hydantoin racemase is a sequence containing a sequence as described in SEQ ID NO:4, or contains a sequence having a homology of at least 90% with the sequence represented by SEQ ID NO:4, and wherein the hydantoin is a hydantoin according to formula [3].or [4]. As depicted in claim 1 and 2 respectively.

4. Use of a whole cell catalytic system according to claim 2 wherein the carbamoylase and the hydantoinase are derived from species other than an *Agrobacterium* species.

5. Use of a whole cell catalytic system according to any one of claims 2-3, **characterized by** the coexpression of hydantoinase of the sequence represented by SEQ ID NO: 2 or by a protein having a homology of at least 90% with the sequence represented by SEQ ID NO:2, carbamoylase, of the sequence represented by SEQ ID NO: 3 or by a protein having a homology of at least 90% with the sequence represented by SEQ ID NO:3 and racemase of the sequence represented by SEQ ID NO: 4 or by a protein having a homology of at least 90% with the sequence represented by SEQ ID NO:4.

6. Use of a Whole cell catalytic system according to any one of claims 2-4, wherein the recombinant microorganism is *E*. *coli.*

7. Method for the preparation of an enantiomerically enriched L-α-amino acid comprising conversion of a hydantoin of formula **[3]** as depicted in claim 1. 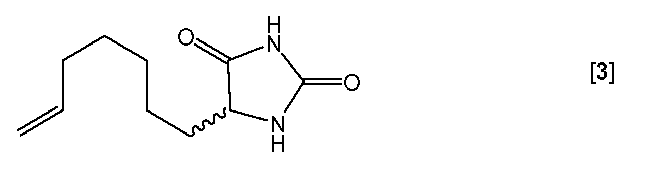 into an L-α-amino acid of general formula [2] 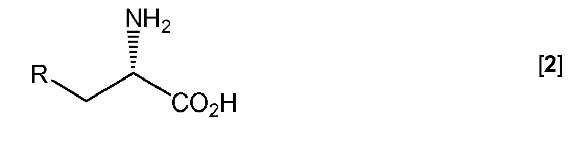 wherein R has the meaning CH₂=CH CH₂ CH₂ CH₂ CH₂-,and wherein a whole cell catalytic system wherein a hydantoinase, L-carbamoylase and hydantoin racemase are coexpressed in a recombinant micro-organism wherein the genes coding for these three enzymes are located on a single replicon, and wherein the hydantoin racemase is derived from an *Agrobacterium* species, and wherein the hydantoin racemase is a sequence containing a sequence as described in SEQ ID NO:4, or contains a sequence having a homology of at least 90% with the sequence represented by SEQ ID NO:4.

8. A method according to claim 6, wherein the carbamoylase and the hydantoinase are derived from species other than an *Agrobacterium* species.

9. A method according to claim 6 or 7, **characterized by** the coexpression of hydantoinase of the sequence represented by SEQ ID NO: 2 or by a protein having a homology of at least 90% with the sequence represented by SEQ ID NO:2, carbamoylase, of the sequence represented by SEQ ID NO: 3 or by a protein having a homology of at least 90% with the sequence represented by SEQ ID NO:3 and racemase of the sequence represented by SEQ ID NO: 4 or by a protein having a homology of at least 90% with the sequence represented by SEQ ID NO:4

10. Method according to any one of claims 6-9 wherein the recombinant microorganism is *E*. *coli.*


SEQUENCE LISTING

<110>  DSM Intellectual Assets B.V.

<120>  Whole cell catalytic system

<130>  25863WO

<150>  EP06025019.8
<151>  2006-12-04

<160>  7

<170>  PatentIn version 3.3

<210>  1
<211>  3423
<212>  DNA
<213>  synthetic


<220>
<221>  CDS
<222>  (15)..(1391)

<220>
<221>  CDS
<222>  (1423)..(2652)

<220>
<221>  CDS
<222>  (2684)..(3400)

<400>  1
aggaggaatt aacc atg ttt gat gta atc gtg aaa aat tgc cgt ctg gta        50
                Met Phe Asp Val Ile Val Lys Asn Cys Arg Leu Val
                1               5                   10

tct tcc gac ggt att acc gaa gca gat atc ctg gtc aag gat ggc aaa        98
Ser Ser Asp Gly Ile Thr Glu Ala Asp Ile Leu Val Lys Asp Gly Lys
        15                  20                  25

gtg gcg gct atc tcc gca gac acc agc gac gtg gaa gcg agc cgt acc       146
Val Ala Ala Ile Ser Ala Asp Thr Ser Asp Val Glu Ala Ser Arg Thr
    30                  35                  40

atc gat gct ggt ggc aag ttc gta atg ccg ggt gta gtg gac gaa cac       194
Ile Asp Ala Gly Gly Lys Phe Val Met Pro Gly Val Val Asp Glu His
45                  50                  55                  60

gtg cac att atc gac atg gac ctg aaa aac cgc tat ggt cgc ttc gaa       242
Val His Ile Ile Asp Met Asp Leu Lys Asn Arg Tyr Gly Arg Phe Glu
                65                  70                  75

ctg gat tcc gaa tct gct gca gtt ggt ggc atc acc acc atc atc gaa       290
Leu Asp Ser Glu Ser Ala Ala Val Gly Gly Ile Thr Thr Ile Ile Glu
            80                  85                  90

atg cca att acc ttc cct ccg acc acc acc ctg gat gcc ttt ctg gaa       338
Met Pro Ile Thr Phe Pro Pro Thr Thr Thr Leu Asp Ala Phe Leu Glu
        95                  100                 105

aag aaa aag cag gca ggt cag cgc ctg aaa gtg gat ttc gca ctg tac       386
Lys Lys Lys Gln Ala Gly Gln Arg Leu Lys Val Asp Phe Ala Leu Tyr
    110                 115                 120

ggc ggt ggt gtg ccg ggt aac ctg ccg gag atc cgt aag atg cac gat       434
Gly Gly Gly Val Pro Gly Asn Leu Pro Glu Ile Arg Lys Met His Asp
125                 130                 135                 140

gca ggt gct gtc ggc ttc aaa agc atg atg gcg gct tcc gtc ccg ggt       482
Ala Gly Ala Val Gly Phe Lys Ser Met Met Ala Ala Ser Val Pro Gly
                145                 150                 155

atg ttc gat gcg gtt tcc gac ggt gaa ctg ttc gaa atc ttt cag gaa       530
Met Phe Asp Ala Val Ser Asp Gly Glu Leu Phe Glu Ile Phe Gln Glu

            160                 165                 170

att gcg gcc tgt ggt agc gtt atc gtg gtt cac gct gaa aac gaa acg 578
Ile Ala Ala Cys Gly Ser Val Ile Val Val His Ala Glu Asn Glu Thr
        175                 180                 185

atc att cag gct ctg cag aaa caa att aag gct gct ggc ggc aag gac 626
Ile Ile Gln Ala Leu Gln Lys Gln Ile Lys Ala Ala Gly Gly Lys Asp
    190                 195                 200

atg gct gct tac gag gcg agc cag cca gtt ttc caa gaa aat gag gcg 674
Met Ala Ala Tyr Glu Ala Ser Gln Pro Val Phe Gln Glu Asn Glu Ala
205                 210                 215                 220

atc cag cgc gct ctg ctg ctg cag aaa gaa gca ggt tgc cgc ctg atc 722
Ile Gln Arg Ala Leu Leu Leu Gln Lys Glu Ala Gly Cys Arg Leu Ile
                225                 230                 235

gtg ctg cac gtt agc aac ccg gac ggt gtg gag ctg atc cat caa gca 770
Val Leu His Val Ser Asn Pro Asp Gly Val Glu Leu Ile His Gln Ala
            240                 245                 250

cag tcc gag ggt cag gac gtt cac tgc gag tct ggc ccg caa tac ctg 818
Gln Ser Glu Gly Gln Asp Val His Cys Glu Ser Gly Pro Gln Tyr Leu
        255                 260                 265

aac atc act acc gat gat gca gaa cgt atc ggt cca tat atg aag gta 866
Asn Ile Thr Thr Asp Asp Ala Glu Arg Ile Gly Pro Tyr Met Lys Val
    270                 275                 280

gcc ccg cca gtc cgt tcc gcc gaa atg aac att cgt ctg tgg gaa caa 914
Ala Pro Pro Val Arg Ser Ala Glu Met Asn Ile Arg Leu Trp Glu Gln
285                 290                 295                 300

ctg gaa aac ggc ctg atc gac acc ctg ggc tct gat cac ggc ggc cac 962
Leu Glu Asn Gly Leu Ile Asp Thr Leu Gly Ser Asp His Gly Gly His
                305                 310                 315

cca gtt gag gac aaa gaa ccg ggc tgg aag gat gtt tgg aag gcg ggt 1010
Pro Val Glu Asp Lys Glu Pro Gly Trp Lys Asp Val Trp Lys Ala Gly
            320                 325                 330

aac ggc gcc ctg ggc ctg gaa act agc ctg ccg atg atg ctg act aac 1058
Asn Gly Ala Leu Gly Leu Glu Thr Ser Leu Pro Met Met Leu Thr Asn
        335                 340                 345

ggt gtt aac aaa ggc cgt ctg tcc ctg gaa cgt ctg gtt gag gtt atg 1106
Gly Val Asn Lys Gly Arg Leu Ser Leu Glu Arg Leu Val Glu Val Met
    350                 355                 360

tgc gaa aaa cca gct aag ctg ttt ggc atc tac ccg cag aaa ggc acc 1154
Cys Glu Lys Pro Ala Lys Leu Phe Gly Ile Tyr Pro Gln Lys Gly Thr
365                 370                 375                 380

ctg cag gtc ggt tct gac gcg gat ctg ctg atc ctg gat ctg gat att 1202
Leu Gln Val Gly Ser Asp Ala Asp Leu Leu Ile Leu Asp Leu Asp Ile
                385                 390                 395

gac acg aag gtc gat gct tct cag ttc cgt tct ctg cac aag tat tct 1250
Asp Thr Lys Val Asp Ala Ser Gln Phe Arg Ser Leu His Lys Tyr Ser
            400                 405                 410

ccg ttc gac ggt atg ccg gta acc ggc gca ccg gta ctg acg atg gta 1298
Pro Phe Asp Gly Met Pro Val Thr Gly Ala Pro Val Leu Thr Met Val
        415                 420                 425

cgt ggt acg gtg gta gcc gaa aaa ggt gaa gtt ctg gtt gag cag ggc 1346
Arg Gly Thr Val Val Ala Glu Lys Gly Glu Val Leu Val Glu Gln Gly
    430                 435                 440

ttc ggc cag ttc gtt act cgt cgt aac tat gaa gca agc aaa taa 1391
Phe Gly Gln Phe Val Thr Arg Arg Asn Tyr Glu Ala Ser Lys
445                 450                 455

atctaagtta tcatatgagg aggaattaac c atg att caa ggt gaa cgt ctg 1443
Met Ile Gln Gly Glu Arg Leu

                                    460                 465

tgg cag cgt ctg atg gaa ctg ggt gaa gtc ggt aaa cag ccg agc ggc      1491
Trp Gln Arg Leu Met Glu Leu Gly Glu Val Gly Lys Gln Pro Ser Gly
                470                 475                 480

ggc gtt act cgc ctg tcc ttc act gcg gaa gaa cgc cgt gcc aaa gac      1539
Gly Val Thr Arg Leu Ser Phe Thr Ala Glu Glu Arg Arg Ala Lys Asp
            485                 490                 495

ctg gtt gcc agc tat atg cgc gaa gct ggt ctg ttt gta tac gaa gac      1587
Leu Val Ala Ser Tyr Met Arg Glu Ala Gly Leu Phe Val Tyr Glu Asp
        500                 505                 510

gca gca ggc aac ctg atc ggc cgt aaa gaa ggc acc aac ccg gat gca      1635
Ala Ala Gly Asn Leu Ile Gly Arg Lys Glu Gly Thr Asn Pro Asp Ala
    515                 520                 525

acc gtg gtt ctg gtt ggt tct cat ctg gac tct gtc tac aac ggc ggt      1683
Thr Val Val Leu Val Gly Ser His Leu Asp Ser Val Tyr Asn Gly Gly
530                 535                 540                 545

tgt ttc gat ggt ccg ctg ggt gtt ctg gct ggc gtt gag gtt gtg cag      1731
Cys Phe Asp Gly Pro Leu Gly Val Leu Ala Gly Val Glu Val Val Gln
                550                 555                 560

act atg aac gaa cac ggt gtg gtc acg cac cac ccg att gaa gtt gtt      1779
Thr Met Asn Glu His Gly Val Val Thr His His Pro Ile Glu Val Val
            565                 570                 575

gcg ttc acc gat gaa gaa ggt gcg cgc ttc cgt ttt ggc atg att ggc      1827
Ala Phe Thr Asp Glu Glu Gly Ala Arg Phe Arg Phe Gly Met Ile Gly
        580                 585                 590

agc cgc gca atg gct ggc acc ctg ccg cct gaa gct ctg gaa tgt cgt      1875
Ser Arg Ala Met Ala Gly Thr Leu Pro Pro Glu Ala Leu Glu Cys Arg
    595                 600                 605

gac gca gaa ggt atc agc ctg gcg gag gct atg aag cag gcg ggt ctg      1923
Asp Ala Glu Gly Ile Ser Leu Ala Glu Ala Met Lys Gln Ala Gly Leu
610                 615                 620                 625

gac cca gat cgt ctg ccg caa gca gca cgc aaa cca ggc acc gtg aaa      1971
Asp Pro Asp Arg Leu Pro Gln Ala Ala Arg Lys Pro Gly Thr Val Lys
                630                 635                 640

gcg tac gtt gaa ctg cac atc gaa cag ggt cgt gta ctg gaa gag gct      2019
Ala Tyr Val Glu Leu His Ile Glu Gln Gly Arg Val Leu Glu Glu Ala
            645                 650                 655

ggt ctg cca gta ggt atc gtg acc ggc att gcg ggt ctg atc tgg gtt      2067
Gly Leu Pro Val Gly Ile Val Thr Gly Ile Ala Gly Leu Ile Trp Val
        660                 665                 670

aaa ttt act att gcg ggc cct gcc gag cac gcc ggt gca acg ccg atg      2115
Lys Phe Thr Ile Ala Gly Pro Ala Glu His Ala Gly Ala Thr Pro Met
    675                 680                 685

agc ctg cgc cgc gac ccg atg gcc gca gct gcc cag atc att atc gtg      2163
Ser Leu Arg Arg Asp Pro Met Ala Ala Ala Ala Gln Ile Ile Ile Val
690                 695                 700                 705

atc gag gaa gaa gct cgt cgt acc ggc acc acc gta ggc acc gtt ggt      2211
Ile Glu Glu Glu Ala Arg Arg Thr Gly Thr Thr Val Gly Thr Val Gly
                710                 715                 720

cag ctg cac gtt tac ccg ggt ggt att aac gtt atc ccg gaa cgt gta      2259
Gln Leu His Val Tyr Pro Gly Gly Ile Asn Val Ile Pro Glu Arg Val
            725                 730                 735

gaa ttc gtg ctg gat ctg cgt gat ctg aag gcc gaa gtt cgt gac caa      2307
Glu Phe Val Leu Asp Leu Arg Asp Leu Lys Ala Glu Val Arg Asp Gln
        740                 745                 750

gtg tgg aaa gcg atc gca gtt cgt gcc gaa acc att gct aaa gaa cgc      2355
Val Trp Lys Ala Ile Ala Val Arg Ala Glu Thr Ile Ala Lys Glu Arg

755                    760                    765

aac gta cgc ctg act acc gaa cgt ctg cag gaa atg gca ccg gtt ctg      2403
Asn Val Arg Leu Thr Thr Glu Arg Leu Gln Glu Met Ala Pro Val Leu
770                 775                 780                 785

tgt tcc gaa gtt gtg aaa caa gcg gca gag cgt gca tgc aaa cag ctg      2451
Cys Ser Glu Val Val Lys Gln Ala Ala Glu Arg Ala Cys Lys Gln Leu
                790                 795                 800

ggc tac ccg ccg ttt tgg ctg cca tct ggc gcg gcc cac gac ggc gtg      2499
Gly Tyr Pro Pro Phe Trp Leu Pro Ser Gly Ala Ala His Asp Gly Val
            805                 810                 815

cag ctg gcg ccg atc tgc ccg atc ggc atg atc ttt gtc cgt agc caa      2547
Gln Leu Ala Pro Ile Cys Pro Ile Gly Met Ile Phe Val Arg Ser Gln
        820                 825                 830

gat ggc gtt tct cac tct ccg gcc gaa tgg tcc acc aaa gaa gac tgc      2595
Asp Gly Val Ser His Ser Pro Ala Glu Trp Ser Thr Lys Glu Asp Cys
    835                 840                 845

gct gtt ggt gcg gag gtt ctg tat cac act gtt tgg cag ctg gct cag      2643
Ala Val Gly Ala Glu Val Leu Tyr His Thr Val Trp Gln Leu Ala Gln
850                 855                 860                 865

ggt gaa taa atctaagtta tggtaccagg aggaattaac c atg cat atc cgt      2695
Gly Glu                                        Met His Ile Arg
                                                        870

ctg att aac ccg aat tct act gcc tct atg acc gca cag gcg ctg gat      2743
Leu Ile Asn Pro Asn Ser Thr Ala Ser Met Thr Ala Gln Ala Leu Asp
            875                 880                 885

agc gca ctg cgc gta aaa cag gca cat acc act atc tct gca act aac      2791
Ser Ala Leu Arg Val Lys Gln Ala His Thr Thr Ile Ser Ala Thr Asn
        890                 895                 900

ccg ctg gac acg ccg gtg tct atc gaa ggc ggt gca gac gaa gca ctg      2839
Pro Leu Asp Thr Pro Val Ser Ile Glu Gly Gly Ala Asp Glu Ala Leu
    905                 910                 915

gct gta ccg ggc atg ctg gaa gaa att cgt aaa ggc gag cgt ctg ggt      2887
Ala Val Pro Gly Met Leu Glu Glu Ile Arg Lys Gly Glu Arg Leu Gly
920                 925                 930                 935

gtc gac gct tac gtg atc gca tgc ttc gac gac ccg ggc ctg cac gct      2935
Val Asp Ala Tyr Val Ile Ala Cys Phe Asp Asp Pro Gly Leu His Ala
                940                 945                 950

gcc cgt gaa gtt gcg cgc ggt ccg gta atc ggt atc tgc cag gcc ggc      2983
Ala Arg Glu Val Ala Arg Gly Pro Val Ile Gly Ile Cys Gln Ala Gly
            955                 960                 965

att cag gta gct atg acc atc agc cgc cgt ttt agc atc atc acg acc      3031
Ile Gln Val Ala Met Thr Ile Ser Arg Arg Phe Ser Ile Ile Thr Thr
        970                 975                 980

ctg ccg cgt tcc atc ccg atc atc gaa gac ctg gtc gat aac tat ggc      3079
Leu Pro Arg Ser Ile Pro Ile Ile Glu Asp Leu Val Asp Asn Tyr Gly
    985                 990                 995

gcg  cag cgt cat tgc cgt  cgt gtc cgt gcc att  aac ctg ccg gtt      3124
Ala  Gln Arg His Cys Arg  Arg Val Arg Ala Ile  Asn Leu Pro Val
1000                 1005                 1010

ctg  ggt ctg gaa gaa gac  ccg cat gct gct gaa  gca atg ctg att      3169
Leu  Gly Leu Glu Glu Asp  Pro His Ala Ala Glu  Ala Met Leu Ile
1015                 1020                 1025

cgc  gag atc gag gcg gcg  aaa aag gaa gac gcc  gcg gag gcg atc      3214
Arg  Glu Ile Glu Ala Ala  Lys Lys Glu Asp Ala  Ala Glu Ala Ile
1030                 1035                 1040

att  ctg ggt tgc gcc ggc  atg tct gct ctg tgc  gat cgt ctg cgc      3259
Ile  Leu Gly Cys Ala Gly  Met Ser Ala Leu Cys  Asp Arg Leu Arg

1045                    1050                    1055

gaa  gcg act ggt gtt ccg  gtg atc gac ggt gtt  acc gcg gca gta       3304
Glu  Ala Thr Gly Val Pro  Val Ile Asp Gly Val  Thr Ala Ala Val
1060                    1065                    1070

aaa  ctg gca gaa gcc ctg  gtg ggc gct ggt tac  agc act agc aaa       3349
Lys  Leu Ala Glu Ala Leu  Val Gly Ala Gly Tyr  Ser Thr Ser Lys
1075                    1080                    1085

gtg  aac gcg tac gat tac  ccg cgt atc aaa gaa  acc aaa ctg gtg       3394
Val  Asn Ala Tyr Asp Tyr  Pro Arg Ile Lys Glu  Thr Lys Leu Val
1090                    1095                    1100

gcc  taa tagcaagttt aggtacccat atg                                   3423
Ala
1105

<210>  2
<211>  458
<212>  PRT
<213>  synthetic

<400>  2

Met Phe Asp Val Ile Val Lys Asn Cys Arg Leu Val Ser Ser Asp Gly
1               5                   10                  15

Ile Thr Glu Ala Asp Ile Leu Val Lys Asp Gly Lys Val Ala Ala Ile
            20                  25                  30

Ser Ala Asp Thr Ser Asp Val Glu Ala Ser Arg Thr Ile Asp Ala Gly
        35                  40                  45

Gly Lys Phe Val Met Pro Gly Val Val Asp Glu His Val His Ile Ile
    50                  55                  60

Asp Met Asp Leu Lys Asn Arg Tyr Gly Arg Phe Glu Leu Asp Ser Glu
65                  70                  75                  80

Ser Ala Ala Val Gly Gly Ile Thr Thr Ile Ile Glu Met Pro Ile Thr
                85                  90                  95

Phe Pro Pro Thr Thr Thr Leu Asp Ala Phe Leu Glu Lys Lys Lys Gln
            100                 105                 110

Ala Gly Gln Arg Leu Lys Val Asp Phe Ala Leu Tyr Gly Gly Gly Val
        115                 120                 125

Pro Gly Asn Leu Pro Glu Ile Arg Lys Met His Asp Ala Gly Ala Val
    130                 135                 140

Gly Phe Lys Ser Met Met Ala Ala Ser Val Pro Gly Met Phe Asp Ala
145                 150                 155                 160

Val Ser Asp Gly Glu Leu Phe Glu Ile Phe Gln Glu Ile Ala Ala Cys
                165                 170                 175

Gly Ser Val Ile Val Val His Ala Glu Asn Glu Thr Ile Ile Gln Ala
            180                 185                 190

Leu Gln Lys Gln Ile Lys Ala Ala Gly Gly Lys Asp Met Ala Ala Tyr
        195                 200                 205

Glu Ala Ser Gln Pro Val Phe Gln Glu Asn Glu Ala Ile Gln Arg Ala
    210                 215                 220

Leu Leu Leu Gln Lys Glu Ala Gly Cys Arg Leu Ile Val Leu His Val
225                 230                 235                 240

Ser Asn Pro Asp Gly Val Glu Leu Ile His Gln Ala Gln Ser Glu Gly
                245                 250                 255

Gln Asp Val His Cys Glu Ser Gly Pro Gln Tyr Leu Asn Ile Thr Thr
            260                 265                 270

Asp Asp Ala Glu Arg Ile Gly Pro Tyr Met Lys Val Ala Pro Pro Val
        275                 280                 285

Arg Ser Ala Glu Met Asn Ile Arg Leu Trp Glu Gln Leu Glu Asn Gly
    290                 295                 300

Leu Ile Asp Thr Leu Gly Ser Asp His Gly Gly His Pro Val Glu Asp
305                 310                 315                 320

Lys Glu Pro Gly Trp Lys Asp Val Trp Lys Ala Gly Asn Gly Ala Leu
                325                 330                 335

Gly Leu Glu Thr Ser Leu Pro Met Met Leu Thr Asn Gly Val Asn Lys
            340                 345                 350

Gly Arg Leu Ser Leu Glu Arg Leu Val Glu Val Met Cys Glu Lys Pro
        355                 360                 365

Ala Lys Leu Phe Gly Ile Tyr Pro Gln Lys Gly Thr Leu Gln Val Gly
    370                 375                 380

Ser Asp Ala Asp Leu Leu Ile Leu Asp Leu Asp Ile Asp Thr Lys Val
385                 390                 395                 400

Asp Ala Ser Gln Phe Arg Ser Leu His Lys Tyr Ser Pro Phe Asp Gly
                405                 410                 415

Met Pro Val Thr Gly Ala Pro Val Leu Thr Met Val Arg Gly Thr Val
            420                 425                 430

Val Ala Glu Lys Gly Glu Val Leu Val Glu Gln Gly Phe Gly Gln Phe
        435                 440                 445

Val Thr Arg Arg Asn Tyr Glu Ala Ser Lys
    450                 455

<210>  3
<211>  409
<212>  PRT
<213>  synthetic

<400>  3

Met Ile Gln Gly Glu Arg Leu Trp Gln Arg Leu Met Glu Leu Gly Glu
1               5                   10                  15

Val Gly Lys Gln Pro Ser Gly Gly Val Thr Arg Leu Ser Phe Thr Ala
            20                  25                  30

Glu Glu Arg Arg Ala Lys Asp Leu Val Ala Ser Tyr Met Arg Glu Ala
        35                  40                  45

Gly Leu Phe Val Tyr Glu Asp Ala Ala Gly Asn Leu Ile Gly Arg Lys
    50                  55                  60

Glu Gly Thr Asn Pro Asp Ala Thr Val Val Leu Val Gly Ser His Leu
65                  70                  75                  80

Asp Ser Val Tyr Asn Gly Gly Cys Phe Asp Gly Pro Leu Gly Val Leu
                85                  90                  95

Ala Gly Val Glu Val Val Gln Thr Met Asn Glu His Gly Val Val Thr
            100                 105                 110

His His Pro Ile Glu Val Val Ala Phe Thr Asp Glu Glu Gly Ala Arg
        115                 120                 125

Phe Arg Phe Gly Met Ile Gly Ser Arg Ala Met Ala Gly Thr Leu Pro
    130                 135                 140

Pro Glu Ala Leu Glu Cys Arg Asp Ala Glu Gly Ile Ser Leu Ala Glu
145                 150                 155                 160

Ala Met Lys Gln Ala Gly Leu Asp Pro Asp Arg Leu Pro Gln Ala Ala
                165                 170                 175

Arg Lys Pro Gly Thr Val Lys Ala Tyr Val Glu Leu His Ile Glu Gln
            180                 185                 190

Gly Arg Val Leu Glu Glu Ala Gly Leu Pro Val Gly Ile Val Thr Gly
        195                 200                 205

Ile Ala Gly Leu Ile Trp Val Lys Phe Thr Ile Ala Gly Pro Ala Glu
    210                 215                 220

His Ala Gly Ala Thr Pro Met Ser Leu Arg Arg Asp Pro Met Ala Ala
225                 230                 235                 240

Ala Ala Gln Ile Ile Ile Val Ile Glu Glu Glu Ala Arg Arg Thr Gly
                245                 250                 255

Thr Thr Val Gly Thr Val Gly Gln Leu His Val Tyr Pro Gly Gly Ile
            260                 265                 270

Asn Val Ile Pro Glu Arg Val Glu Phe Val Leu Asp Leu Arg Asp Leu
        275                 280                 285

Lys Ala Glu Val Arg Asp Gln Val Trp Lys Ala Ile Ala Val Arg Ala
    290                 295                 300

Glu Thr Ile Ala Lys Glu Arg Asn Val Arg Leu Thr Thr Glu Arg Leu
305                 310                 315                 320

Gln Glu Met Ala Pro Val Leu Cys Ser Glu Val Val Lys Gln Ala Ala
                325                 330                 335

Glu Arg Ala Cys Lys Gln Leu Gly Tyr Pro Pro Phe Trp Leu Pro Ser
            340                 345                 350

Gly Ala Ala His Asp Gly Val Gln Leu Ala Pro Ile Cys Pro Ile Gly
        355                 360                 365

Met Ile Phe Val Arg Ser Gln Asp Gly Val Ser His Ser Pro Ala Glu
    370                 375                 380

Trp Ser Thr Lys Glu Asp Cys Ala Val Gly Ala Glu Val Leu Tyr His
385                 390                 395                 400

Thr Val Trp Gln Leu Ala Gln Gly Glu
                405

<210>  4
<211>  238
<212>  PRT
<213>  synthetic

<400>  4

Met His Ile Arg Leu Ile Asn Pro Asn Ser Thr Ala Ser Met Thr Ala
1               5                   10                  15

Gln Ala Leu Asp Ser Ala Leu Arg Val Lys Gln Ala His Thr Thr Ile
            20                  25                  30

Ser Ala Thr Asn Pro Leu Asp Thr Pro Val Ser Ile Glu Gly Gly Ala
        35                  40                  45

Asp Glu Ala Leu Ala Val Pro Gly Met Leu Glu Glu Ile Arg Lys Gly
    50                  55                  60

Glu Arg Leu Gly Val Asp Ala Tyr Val Ile Ala Cys Phe Asp Asp Pro
65                  70                  75                  80

Gly Leu His Ala Ala Arg Glu Val Ala Arg Gly Pro Val Ile Gly Ile
                85                  90                  95

Cys Gln Ala Gly Ile Gln Val Ala Met Thr Ile Ser Arg Arg Phe Ser
            100                 105                 110

Ile Ile Thr Thr Leu Pro Arg Ser Ile Pro Ile Ile Glu Asp Leu Val
        115                 120                 125

Asp Asn Tyr Gly Ala Gln Arg His Cys Arg Arg Val Arg Ala Ile Asn
    130                 135                 140

Leu Pro Val Leu Gly Leu Glu Glu Asp Pro His Ala Ala Glu Ala Met
145                 150                 155                 160

Leu Ile Arg Glu Ile Glu Ala Ala Lys Lys Glu Asp Ala Ala Glu Ala
                165                 170                 175

Ile Ile Leu Gly Cys Ala Gly Met Ser Ala Leu Cys Asp Arg Leu Arg
            180                 185                 190

Glu Ala Thr Gly Val Pro Val Ile Asp Gly Val Thr Ala Ala Val Lys
        195                 200                 205

Leu Ala Glu Ala Leu Val Gly Ala Gly Tyr Ser Thr Ser Lys Val Asn
    210                 215                 220

Ala Tyr Asp Tyr Pro Arg Ile Lys Glu Thr Lys Leu Val Ala
225                 230                 235

<210>  5
<211>  14
<212>  DNA
<213>  synthetic

<220>
<221>  misc_feature
<222>  (1)..(14)

<400>  5
aggaggaatt aacc                                                         14

<210>  6
<211>  31
<212>  DNA
<213>  synthetic

<220>
<221>  misc_feature
<222>  (1)..(31)

<400>  6
atctaagtta tcatatgagg aggaattaac c                                      31

<210>  7
<211>  23
<212>  DNA
<213>  synthetic

<220>
<221>  misc_feature
<222>  (1)..(23)

<400>  7
tagcaagttt aggtacccat atg                                               23